# EUROPEAN PATENT SPECIFICATION

(11) **EP 2 303 200 B1**
(45) Date of publication and mention of the grant of the patent: **16.01.2013**
(21) Application number: 09733888.3
(22) Date of filing: 22.04.2009
(51) Int. Cl.: A61F 5/00

(54) **REMOTELY ADJUSTABLE GASTRIC BANDING SYSTEM**
FERNEINSTELLBARES MAGENBANDSYSTEM
SYSTÈME DE CERCLAGE GASTRIQUE AJUSTABLE À DISTANCE

(30) Priority: 23.04.2008 US 47356 P
(43) Date of publication of application: 06.04.2011
(73) Proprietor: Allergan, Inc., Irvine, CA 92612 (US)
(72) Inventor: BIRK, Janel, A., Oxnard CA 93036 (US); SNOW, Sean, Carpinteria CA 93013 (US)
(74) Representative: HOFFMANN EITLE
(86) International application number: PCT/US2009/041437
(87) International publication number: WO 2009/132127

(56) References cited:
- EP-A- 1 600 183
- EP-A- 1 602 346
- EP-A- 1 736 123
- US-A1- 2007 156 013

## Description

### Field of the Invention

The present description generally relates to medical systems and apparatus for treating obesity and/or obesity-related diseases, and more specifically, relates to remotely adjustable gastric banding systems.

### Background

Adjustable gastric banding apparatus have provided an effective and substantially less invasive alternative to gastric bypass surgery and other conventional surgical weight loss procedures. Despite the positive outcomes of invasive weight loss procedures, such as gastric bypass surgery, it has been recognized that sustained weight loss can be achieved through a laparoscopically-placed gastric band, for example, the LAP-BAND® (Allergan, Inc., Irvine, CA) gastric band or the LAP-BAND AP® (Allergan, Inc., Irvine, CA) gastric band. Generally, gastric bands are placed about the cardia, or upper portion, of a patient's stomach forming a stoma that restricts food's passage into a lower portion of the stomach. When the stoma is of an appropriate size that is restricted by a gastric band, food held in the upper portion of the stomach provides a feeling of satiety or fullness that discourages overeating. Unlike gastric bypass procedures, gastric band apparatus are reversible and require no permanent modification to the gastrointestinal tract.

Over time, a stoma created by a gastric band may need adjustment in order to maintain an appropriate size, which is neither too restrictive nor too passive.
Accordingly, prior art gastric band systems provide a subcutaneous fluid access port connected to an expandable or inflatable portion of the gastric band. By adding fluid to or removing fluid from the inflatable portion by means of a hypodermic needle inserted into the access port, the effective size of the gastric band can be adjusted to provide a tighter or looser constriction.

Naturally, it would be desirable to allow for non-invasive adjustment of gastric band constriction, for example, without the use of a hypodermic needle. Thus, remotely adjustable gastric banding systems have been proposed and are described herein.
EP 1 600 183 A relates to a remotely controlled gastric band system that is intended to be immune to external magnetic fields.
US 2007/156013 A1 relates to a self-regulating gastric band apparatus for adjusting stoma size.
EP 1 602 346 A relates to an implantable adjustable sphincter system.
EP 1 736 123 A relates to a remote monitoring and adjustment of food intake restriction device that can be implanted within a patient.

### SUMMARY

Generally described herein are remotely adjustable and powered gastric band systems. The systems described herein aid in facilitating obesity control and/or treating obesity-related diseases while being non-invasive once implanted.

The systems generally comprise a gastric band having at least one inflatable member or bladder and a high precision pump unit in communication with or couplable to at least one inflatable member of the gastric band.

The present invention relates to a system for facilitating obesity control as defined in independent claim 1. Advantageous further embodiments are recited in the dependent claims.

The high precision pump unit described herein is an implantable, high precision pump device capable of adjusting, and even fine tuning, the stoma created by the gastric band. In some embodiments, high precision pump unit may be substantially passive and requires minimal power to operate. Further, the high precision pump unit is configured and structured to enable tightening and/or loosening of a gastric band by moving a metered amount of fluid between an implantable reservoir and the at least one inflatable member of the gastric band. The high precision pump unit can also be controlled by a handheld remote control unit operated by a clinician, patient or caretaker.

In one example embodiment, the high precision pump unit comprises at least one high precision piezoelectric, unidirectional pump. The pump is unidirectional in that it allows flow only in a direction from the reservoir to the at least one inflatable member or bladder of the gastric band. The pump is effective in pumping small, metered volumes of fluid into the at least one inflatable member. At least one check valve may be provided within the high precision pump unit for preventing flow in an opposing direction of the desired flow.

Additionally, high precision pump unit further includes a parallel flow line for allowing fluid flow in a direction from the at least one inflatable member to the reservoir, thus decreasing the constriction of the at least one inflatable member against the stoma.

In one example embodiment described herein is a system for facilitating obesity control comprising: a gastric banding device including at least one inflatable member for containing fluid; a fluid reservoir; an implantable pump unit in communication with the fluid reservoir for controlling pressure within the at least one inflatable member, the implantable pump unit including a metering assembly and a receiver assembly in communication with the metering assembly; and a remote control device capable of communicating with the receiver assembly.

In another example embodiment, the metering assembly comprises a piezoelectric pump, or alternatively a piezoelectric diaphragm pump or unidirectional pump. The pump, in some embodiments comprises a flexible membrane. In yet another example embodiment, the piezoelectric pump is effective in drawing fluid away from the reservoir and toward the at least one inflatable member. Alternatively, the piezoelectric pump further comprises a valve effective to pass fluid in a direction from the at least one inflatable member to the reservoir.

In a further example embodiment, the system further comprises a pressure sensor effective in sensing a pressure of the at least one inflatable member or an access port for enabling manual adjustment of a volume or a pressure of fluid in the at least one inflatable member.

In still further example embodiments, the access port is an integral part of the pump unit and implantable pump unit does not require a battery to operate. Further still, in other example embodiments, the system comprises substantially no implanted ferromagnetic materials and is MRI safe.

According to the invention, the pump unit comprises a first fluid line including a one-way pump for passing fluid in a first direction and a second fluid line in parallel with the first line including a valve for passing fluid in an opposing direction.

In one example embodiment of the invention, an implantable high precision pump unit for use with a gastric band is provided. The pump unit generally comprises a inductively powered, piezoelectric pump, a first valve in line with the pump, a second valve in parallel with the pump; electronics in communication with the pump and first and second valves; an override port; and a housing containing the pump, first and second valves, electronics and override port, wherein the pump unit is operable without the use of an implanted energy storage device.

In another embodiment, the high precision pump unit is controlled by an external remote control unit. The high precision pump unit can be inductively powdered by an external remote control unit in one example embodiment.

In still another example embodiment, the housing of the high precision pump unit is hermetically sealed. In yet another example embodiment, the override port is manufactured at a position that is located away from at least one tube emanating from the high precision pump unit. Even further still, in another example embodiment, the at least one pump can deliver fluid to at least one gastric band from at least one reservoir, both connected to the high precision pump unit via at least one tube.

In one example embodiment described herein is a system for facilitating obesity control comprising: an implantable gastric banding device including at least one inflatable member for containing fluid and restricting a patient's cardia; an implantable fluid reservoir; an implantable pump unit in communication with the fluid reservoir and the gastric band device for controlling pressure within the at least one inflatable member, the implantable pump unit including at least one piezoelectric pump and at least one valve in a parallel configuration and a receiver in direct communication with at least one piezoelectric pump and at least one valve; and an external remote control device capable of communicating with the receiver assembly and powering the implantable pump unit. In another example embodiment, the system further comprises an override port located on the implantable pump unit.

### BRIEF DESCRIPTION OF THE DRAWINGS

Figure 1 illustrates an overall schematic view of an example configuration of components according to the present description.

Figure 2 illustrates en example configuration of the internals of a high precision pump unit.

Figures 3A and 3B illustrate cross-sections of an example pump.

Figures 4A and 4B illustrate cross-sections of an example valve.

Figures 5A and 5B illustrate cross-sections of an example pressure sensor.

Figure 6 illustrates the filling of a gastric band using the systems described herein.

Figure 7 illustrates the draining of a gastric band using the systems described herein.

### DETAILED DESCRIPTION

The present invention generally provides remotely adjustable gastric banding systems, for example, for treatment of obesity and obesity related conditions, as well as systems for controlling inflation of gastric banding systems.

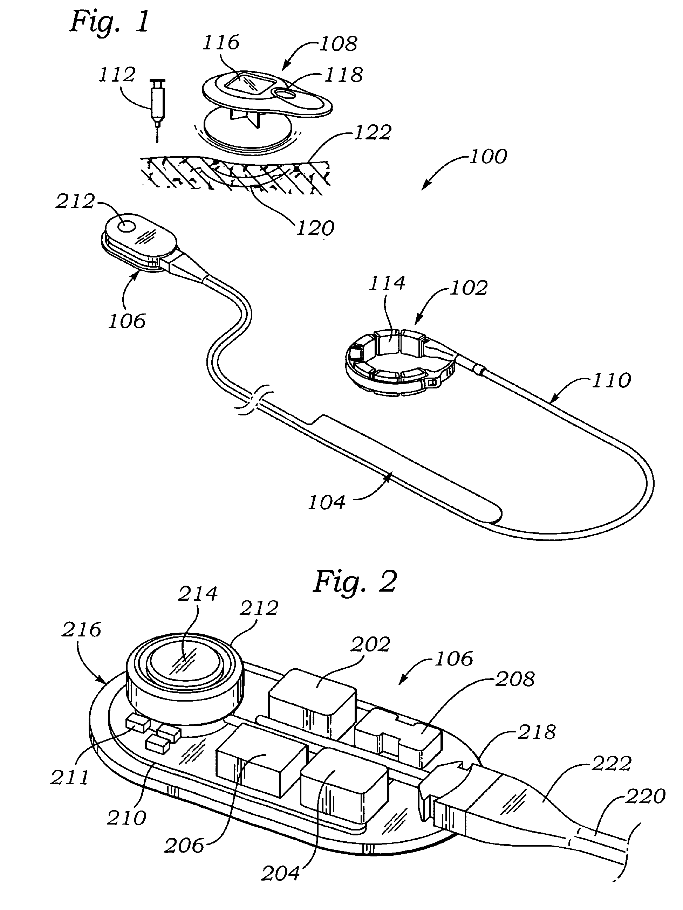

Turning now to Figure 1, a gastric banding system **100** in accordance with one embodiment of the invention generally includes a gastric band **102,** reservoir **104,** high precision pump unit **106,** remote controller unit **108** and tubing **110.** Each of the components of system **100,** other than remote controller unit **108,** is implantable in a patient using conventional surgical techniques. High precision pump unit **106** can be used to replace or complement a conventional access port for adjusting inflation of gastric band **102.** In some embodiments, the system includes an override port 112 which can be used, for example, with a hypodermic needle **112,** to fill and drain the gastric band **102.**

High precision pump unit **106** is connected to reservoir **104** and gastric band **102** via tubing **110,** and can move precisely metered volumes of saline in or out of gastric band **102.** Moving saline into gastric band **102** causes inflation of at least one bladder, or inflatable member **114** and constricts around the cardia, or upper portion of the stomach, forming a stoma that restricts the passage of food into a lower portion of the stomach. This stoma can provide a patient with a sensation of satiety or fullness that discourages overeating. In contrast, moving saline out of at least one inflatable member **114** of gastric band **102** contracts the pressure around the cardia and allows a stoma to be at least partially released and regains the patient's hunger sensation.

High precision pump unit **106** is implanted within a patient, and therefore, is non-biodegradable. The encasement of the unit is hermetically sealed from the *in situ* environment and formed at least partially of any rugged plastic material including, polypropylene, cyclicolephin co-polymer, nylon, and other compatible polymers and the like or at least partially formed of a non-radioopaque metal such as titanium. The encasement has a smooth exterior shape, with no jagged edges, to minimize foreign body response and tissue irritation. The unit itself is also sterilizable, preferably dry heat sterilizable before implantation.

The internal components of high precision pump unit **106** are illustrated in Figure 2. The encasement of high precision pump unit **106** has an internal volume of less than about 12.3 cm³ (0.75 in³), preferably less than about 8 cm³ (0.5 in³). Exemplary internal features of high precision pump unit **106** that fit within the encasement include first valve **202,** second valve **204,** pump **206,** pressure/flow sensor **208,** electronics board **210** including an antenna **211,** and override port **212.** The internal components of high precision pump unit **106** can be arranged in any fashion appropriate for delivering and removing precise amounts of fluid from gastric band **102** and reservoir **104.**

Pump **206** can be actively or passively driven. If pump **206** is actively driven, a local power source such as a battery (not illustrated) is provided to drive pump **206.** If pump **206** is passively driven, it may be inductively powered by a device external to high precision pump unit **106.** In an exemplary configuration, pump **206** is passively driven through inductive power from remote controller unit **108.**

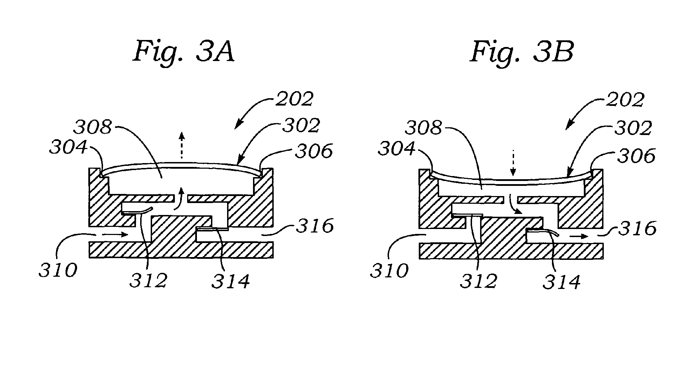

In one example embodiment, pump **206** is an inductively powered, electrically driven, positive displacement piezoelectric pump. Pump **206** provides the means to move fluid into gastric band **102.** With reference to Figure 3A, when a pumping sequence is initiated to move fluid into at least one inflatable member **114** of gastric band **102,** piezoelectric bending membrane **302** is charged and is deflected upwards. Since piezoelectric bending membrane **302** is sealed along first edge **304** and second edge **306,** a negative pressure is created which pulls fluid into chamber **308** from inlet line **310.** First check valve **312** opens to permit flow while second check valve **314** remains closed.

Then, referring to Figure 3B, when electric potential is reversed to pump **202,** piezoelectric bending membrane **302** deflects downward and forces fluid out of chamber **308.** As fluid is forced out of chamber **308,** first check valve **312** is forced closed while second check valve **314** opens forcing fluid out of exit line **316.** In a preferred embodiment, inlet line **310** is connected either directly or indirectly to reservoir **104** and exit line **316** is connected either directly or indirectly to gastric band **102.**

Pump **202** can move fluid from the reservoir **104** to gastric band **102** at rates higher than about 0.5 cm³/min, for example, higher than about 1 cm³/min for band pressures less than about 138 kPa (about 20 psi) relative to the reservoir pressure. Alternatively, fluid can be drained from gastric band **102** to reservoir **104** at rates higher than about 0.5 cm³/min, for example, higher than about 1 cc/min for band pressures above about 1.38 kPa (about 0.2 psi).

Reservoir **104** is a soft, collapsible balloon made of a biocompatible polymer material, for example, silicone, which holds a reserve of a biocompatible fluid, for example, saline, to allow for adjustments in the size of gastric band **102.** The reservoir is preferably fully collapsible and can contain the extra fluid required to increase the volume of gastric band **102** to therapeutic levels. Further, reservoir **104** also may have excess capacity so gastric band **102** may be fully drained into it without reservoir **104** being filled beyond its maximum capacity.

The fluids used within the systems of the present description include any fluid that is biocompatible. The fluid has no adverse effect on the patient in the unlikely event that a leak emanates from the system. The fluid can simply be water or any biocompatible polymer oil such as caster oil. In an example embodiment, the fluid is saline.

Tubing **110** is any biocompatible flexible tubing that does not degrade *in vivo.* Tubing **110** is configured to withstand hydraulic forces up to about 206 kPa (about 30 psi) without leakage. This hydraulic pressure tolerance is true of the entire fluid path of the systems described herein. Although the systems described herein do not generally leak, if they do, fluid is not lost at a rate greater than about 0.2 cm³/yr, or about 0.1 *cm³*/*yr.*

Other biocompatible and biostable polymers which are useful for forming reservoir **104** and tubing **110** include polyolefins, polyisobutylene and ethylene-alphaolefin copolymers; acrylic polymers and copolymers, ethylene-co-vinylacetate, polybutylmethacrylate, vinyl halide polymers and copolymers, such as polyvinyl chloride; polyvinyl ethers, such as polyvinyl methyl ether; polyvinylidene halides, such as polyvinylidene fluoride and polyvinylidene chloride; polyacrylonitrile, polyvinyl ketones; polyvinyl aromatics, such as polystyrene, polyvinyl esters, such as polyvinyl acetate; copolymers of vinyl monomers with each other and olefins, such as ethylene-methyl methacrylate copolymers, acrylonitrile-styrene copolymers, ABS resins, and ethylene-vinyl acetate copolymers; polyamides, such as Nylon 66 and polycaprolactam; alkyd resins; polycarbonates; polyoxymethylenes; polyimides; polyethers; epoxy resins, polyurethanes; rayon; rayon-triacetate; cellulose, cellulose acetate, cellulose butyrate; cellulose acetate butyrate; cellophane; cellulose nitrate; cellulose propionate; cellulose ethers; and carboxymethyl cellulose.

First valve **202** and second valve **204,** illustrated in Figure 2, can be any valve known in the art to allow precise delivery of fluid and precise flow rates therethrough. Preferably, first valve **202** and second valve **204** only allow fluid to move in one direction, therefore, the two valves are situated in parallel with high precision pump unit **106** allowing fluid to drain back from gastric band **102.** Further, first valve **202** and second valve **204** should have a precision orifice that restricts the flow rate to a well-characterized, precise amount.

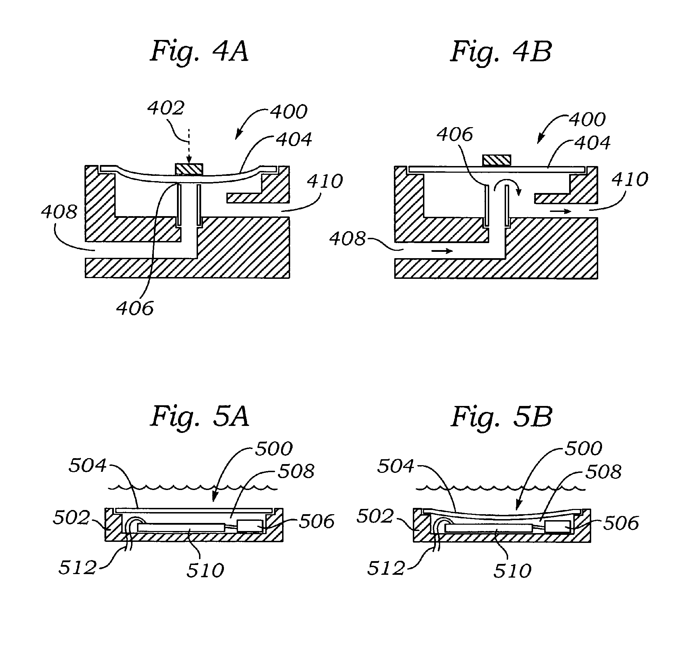

Figures 4A and 4B illustrate an exemplary valve **400** according to an embodiment of the invention. Valve 400 may be used in place of one or both of first valve **202** and second valve **204.**

Referring to Figure 4A, valve **400** is biased in a closed position, for example, by spring preload force **402** acting on a seal **404,** for example, a flexible silicone seal **404.** For example, spring preload force **402** pushes flexible silicone seal **404** into sealing engagement with a valve seat **406.** When valve **400** is sealed as shown in Fig. 4A, fluid cannot pass from valve inlet **408** to valve exit **410.** Now referring to Figure 4B, when flow is desired, a signal is sent to valve actuator (not shown) which removes spring preload force **402** and permits flexible silicone seal **404** to relax into an open position, out of sealing engagement with valve seat **406.** Fluid is then free to flow from valve inlet **408** to valve exit **410** until valve **400** is closed, for example, by reapplication of spring preload force **402.**

Turning back now to Figs. 2, 6 and 7, the system **100** may further comprise at least one flow or pressure sensor **208** disposed, for example, within high precision pump unit **106.** In an exemplary embodiment, two pressure sensors are situated within the fluid pathway between first valve **202** and second valve **204** and gastric band **102.** During a no-flow condition, both of the pressure sensors may be used to measure pressure thereby providing the benefits of redundancy and averaging.

For example, sensing or measuring the pressure within the fluid pathway of system **100** provides diagnostic uses. Clinicians can measure pressure while a patient drinks water, recording and analyzing resulting pressure fluctuations which can help determine if gastric band **102** is too restrictive. A band that is too restrictive can also be confirmed by the patient's response (generally discomfort) upon drinking the water, and can then be appropriately adjusted. Further, sensing or measuring pressure in the system **100** can be useful in diagnosing system leaks or obstructions. For example, if the pressure consistently drops over an extended period of time, the clinician can diagnose a leak within the system and plan for an appropriate treatment to fix the problem. In contrast, if there is an obstruction within the system with a sustained pressure rise over time, the clinician can diagnose an obstruction within the system and plan for an appropriate treatment to fix the problem.

Figures 5A and 5B illustrate an exemplary pressure sensor **500** according to an embodiment of the present invention which can be used in place of pressure sensor **208** in the system **100.** Referring to Figure 5A, pressure sensor **500** is hermetically sealed and comprises housing **502,** for example, a metallic housing, and membrane **504,** both of which may comprise titanium or other non-radioopaque material. Membrane **504** flexes in response to a signal, which affects pressure transducer **506.** Space inside housing **502** may contain degassed silicone oil **508** which serves as an incompressible pressure transfer medium. As pressure outside pressure sensor **500** is increased, as illustrated in Figure 5B, membrane **504** deflects downward increasing the pressure of the degassed silicone oil **508.** Pressure transducer **506** converts pressure changes into changes in capacitance which are then detected by electronics board **510.** Electronics board **510** converts the sensitive analog pressure signal into a hardy digital signal that can pass through housing **502** via signal wire **512** which is substantially immune to typical levels of electrical noise.

Override port **212,** illustrated in Figure 1 and Figure 2 is an optional feature of some embodiments of the present invention. Override port **212** can be manufactured from any metal, preferably titanium or another non-radioopaque and is accessible by insertion of non-coring, hypodermic needle **112** (Figure 1) through a self-sealing septum **214.** Override port **212** allows a clinician to use hypodermic needle **112** or a standard syringe to fill or drain gastric band **102.** Further, override port **212** may be located on distal end **216** of high precision pump unit **106,** for example, at a position substantially opposite from proximal end **218** where tubing **220** extends from high precision pump unit **106.** This placement of override port **212** thereby reduces possible occurrences of a needle damaging tubing **220.** Extension body **222** emanating from high precision pump unit **106** further protects tubing **220** from accidental needle sticks

The systems and apparatus described herein further include remote controller unit **108,** which provides access to system data and functions and is an external, handheld, reusable battery-powered device. Remote controller unit **108** can be made of any rugged plastic material including, polypropylene, cyclicolephin co-polymer, nylon, and other compatible polymers and the like. The controller unit is not implanted within the patient so hermetic sealing of the unit is not required. However, remote controller unit **108** is preferably at least water resistant, if not waterproof, and can be cleaned using standard hospital disinfectants without damage to the unit.

Further, remote controller unit **108** has a user interface including at least one display **116** and at least one user input **118.** In some example embodiments, display **116** and user input **118** are combined in the form of a touch screen with a color display. In other embodiments, the display is grayscale. Remote controller unit **108** permits a clinician or a patient to navigate through menu driven screens used for data entry, data collection, and high precision pump unit **106** control.

Remote controller unit **108** is capable of communicating with high precision pump unit **106.** "Capable of communicating" as used herein refers to the remote controller's ability to establish communications with high precision pump unit **106** yet still have the ability to break communication and the systems described herein still function. To establish communication, in one example embodiment, once remote controller unit **108** is initialized, display **116** shows a searching query for a nearby high precision pump unit **106.** As remote controller unit **108** is brought into range of high precision pump unit **106,** a symbol displays the strength of the communication link. Once stable communications have been acquired, display **116** shows the serial number of the system so a clinician can verify they have the appropriate patient records in hand. If the patient requires a tightening of gastric band **102,** the clinician can enter the amount of the desired volume increase. Remote controller unit **108** can also display the current volume within gastric band **102** and indicate the new volume as gastric band **102** fills. Display **116** can also indicate desired and actual volumes during gastric band **102** draining.

To verify the appropriate adjustment has been made to the system, the clinician can set remote controller unit **108** into pressure monitor mode and request that the patient drink water. Display **118** shows a real time graph of the pressure measured within gastric band **102.** This diagnostic tool may show higher pressures and warning messages if gastric band **102** has been over-tightened.

Remote controller unit **108** can synchronize and charge when coupled with a charging cradle or docking station. This docking station provides the ability to recharge remote controller unit's **108** rechargeable battery and provides a link to download information to a personal computer such as the adjustment history of a patient. Other data that can be stored on remote controller unit **108** and downloaded from high precision pump unit **106** includes, but is not limited to serial number, gastric band size, patient information, firmware version and patient adjustment history. This data can be downloaded directly to a patient tracking database for ease tracking.

Any data stored on remote controller unit **108** or within high precision pump unit **106** can be electronically secured. In other words, security measures can be put in place to keep the data confidential, including communication between high precision pump unit **106** and remote controller unit **108.** Security measures can include computer generated algorithms that prevent intrusion by outside parties.

High precision pump unit **106** can contain a micro-fluidic pump with active valves. In such an embodiment, high precision pump unit **106** is a passive device that can only be powered by remote controller unit **108** when it is in close proximity. For example, in one example embodiment, remote controller unit **108** may be configured to power and communicate with high precision pump unit **106** at any distance less than about 20.3 cm (about 8 inches), preferably less than about 10.2 cm (about 4 inches) of tissue plus about 4 inches, preferably about 5.1 cm (about 2 inches) of air. Power and communications can be tailored to transmit over longer distances or can be tailored to have remote controller unit **108** placed on the skin adjacent to the high precision pump unit **106.**

Further, remote controller unit **108** can inductively power and telemetrically control high precision pump unit **106.** Remote controller unit **108** may be configured to provide continuous power to high precision pump unit **106.** A dedicated microcontroller within remote controller unit **108** monitors the amount of power that is transmitted. Further still, a power management system may be implemented to optimize energy transmission between remote controller unit **108** and high precision pump unit **106** relative to their separation distance. For example, the power transmission may automatically decrease as remote controller unit **108** is closer to high precision pump unit **106,** and may be increased as the distance is increased. This minimizes wasted energy, and energy exposure to the patient.

High precision pump unit **106** is a passive device which may be entirely controlled and powered by remote controller unit **108.** Antenna **211** on electronics board **210** housed within high precision pump unit **106** and remote controller unit **108** are coupled to allow the transmission of power **122** through skin **124** (as illustrated in Figure 1). The power issued from remote controller unit **108** is continually monitored by a dedicated microprocessor to ensure that power transmission is minimized to the lowest level required for operation. To minimize the power transmission and to optimize command communication, high precision pump unit **106** and remote controller unit **108** have a channel frequency dedicated to command communication and a separate channel frequency dedicated to power transmission. The command communication can be configured, for example, to take place at about 402 - 406 MHz while the power transmission, for example, takes place at about 400 kHz. This command communication adheres to the frequency and power standards set by the Medical Implant Communications Service. To ensure accuracy, communication and control commands are verified by error check algorithms prior to data reporting or command implementation.

A portion of electronics board **210** within high precision pump unit **106** is devoted to conditioning and managing the power received at antenna **211** or from a local battery. Communication electronics manage the bidirectional transmissions with timing verification and error checking. Controller circuits of electronics board **210** send commands to first valve **202,** second valve **204,** pump **206,** and pressure/flow sensor **208** and receive data back from pressure/flow sensor **208.** Electronics board **210** can be encased in a biocompatible sealant if further protection, or redundant protection, is necessary.

The systems and apparatus described herein use common surgical techniques to place the components in their respective positions within a patient. The surgical techniques may be identical or similar to those used in the placement of conventional gastric banding systems. For example, gastric band **102** may be placed around the stomach using laparoscopic techniques, as known to those of skill in the art. Like a conventional access port, high precision pump unit **106** may be sutured onto the rectus muscle sheath or any other conveniently accessible muscle. In order to achieve a secure attachment of high precision pump unit **106,** the unit shall be sutured to the rectus muscle and remain securely attached for forces below about 26.7 N (about 6 Ibf), preferably below about 13.3 N (3 lbf). Tubing **110** from high precision pump unit **106** passes through the rectus muscle into the peritoneal cavity in the same manner as the tubing of a conventional access port.

The systems and apparatus of the present description further allows for a remotely controlled adjustment without needles, non-invasively, by using remote controller unit **108.** Also, should remote controller unit **108** be unavailable, damaged, out of power, or in the even of an emergency, an adjustment of gastric band **102** can be performed invasively using a needle. By using override port **212,** a clinician can choose to use a standard needle for adjustments. If any of the electronics associated with the systems and apparatus described herein become inoperable, override port **212** can be used to add or remove fluid from gastric band **102.** Override port **212** and a syringe or needle can always be used to adjust gastric band **102.**

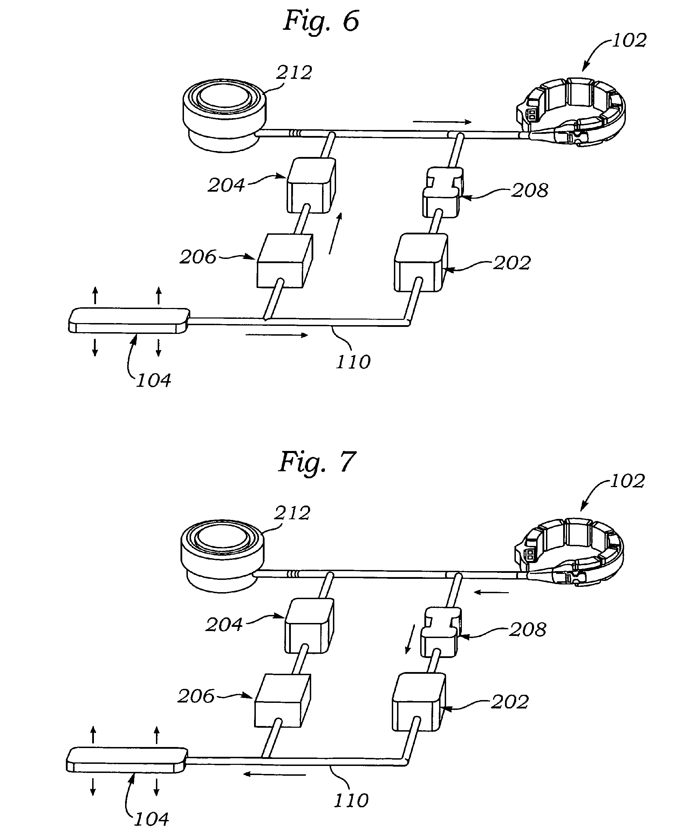

The systems described herein generally function as follows. When a clinician uses remote controller unit **108** to adjust gastric band **102,** high precision pump unit **106** initiates a sequence of events to move a precise amount of fluid in the desired direction. Referring to Figure 6, the system diagram illustrates the filling of gastric band **102.** Just before pumping is initiated, second valve **204** in line with pump **206** is opened. Pump **206** is activated pumping fluid into gastric band **102** which creates a differential pressure to draw fluid out of reservoir **104.** First valve **202** and pressure/flow sensor **208** are not engaged. Reservoir **104** is collapsible and does not impede the outward flow of fluid. Further, reservoir **104** is sized such that when filled to the maximum recommended fill volume, there is a slight vacuum therein. Once the proper amount of fluid has been transferred from reservoir **104** to gastric band **102,** electronics board **210** shuts off pump **206** and closes second valve **204.** Gastric band **102** now assumes the new higher pressure.

Alternatively, if the clinician decides to loosen gastric band **102,** fluid is released from gastric band **102** and returned to reservoir **104.** This process is illustrated in Figure 7. Once high precision pump unit **106** receives a drain command from remote controller unit **108,** first valve **202** behind pressure/flow sensor **208** opens. The amount of fluid released from gastric band **102** is controlled by the amount of time pressure/flow sensor **208** remains open. This timing is based on information from pressure/flow sensor **208** indicating the rate of fluid flow. Once the correct volume of fluid has been transferred, first valve **202** is closed. With both first valve **202** and second valve **204** closed, the volume in gastric band **102** is maintained and the pressure in gastric band **102** can be measured accurately.

When compared to conventional gastric banding systems having standard access ports which exclusively require syringe access, the presently described systems and apparatus offer several benefits. First, for conventional access ports located under a thick layer of fatty tissue, which is generally the case as the devices are generally used to treat obesity, the access port can be difficult to locate. The present systems reduce or eliminate the need for port location as the use of remote controller unit **108** removes the need for adjustment using a syringe.

Secondly, accessing the access port in conventional systems, when there is ambiguity on its location, can cause damage by accidentally puncturing the tubing which connects the access port to the gastric band. This can require a revision surgery in order to repair the punctured tubing. Further, when a conventional access port cannot be located by palpation, x-ray imaging may be required to guide a needle into the access port. Such imaging practices put a patient at risk for x-ray radiation exposure. The present systems and apparatus remove the need for these unnecessary procedures and save the patient from x-ray radiation exposure. As described infra, the present systems and apparatus are compatible with magnetic resonance imaging (MRI), which is much safer for a patient.

In the unlikely event that override port **212** of the present description is used, it may be located away from the tubing connection to gastric band **102** to reduce the potential for tubing needle sticks. High precision pump unit **106** has geometry and a rigid case that can be structured to facilitate the user in locating override port **212** when needed.

In one example embodiment, the systems and apparatus described herein are configured and structured to be compatible with MRI, or MRI safe, at, for example 1.5 T. In the exemplary embodiment shown, the pump unit is entirely inductively powered. The systems utilize no permanent magnets, no long metallic wires or leads, and a minimal or negligible amount of ferrous or ferromagnetic material. The systems are substantially free or contain substantially no ferromagnetic materials. Substantially no ferromagnetic materials refers to materials containing less than about 5%, preferably less than about 1% or 0.1% (w/w) of ferromagnetic material. The resulting systems are thus MRI safe given standard specifications regulating translational and rotational attraction, MRI heating, and imaging artifacts. All materials selected for the systems are preferably selected to be compatible and safe in an MRI environment.

Further, the inductive powering of high precision pump unit **106** requires that energy be passed through body tissue. Since the tissue absorbs a small amount of the energy passing through it, the heating of the tissue can be proportional to the total energy transferred. To ensure that the systems meet standards to minimize tissue heating (below 2°C above body temperature per ISO 45502), the systems described herein have been designed to use very little power to move the fluid within the system and do not cause excessive heating of the patient's tissue.

The systems may also include a pressure sensor to monitor pressure inside gastric band **102** as needed. Using remote controller unit **108** to communicate with high precision pump unit **106,** a clinician can monitor pressure inside gastric band **102,** for example, in "real time" during an adjustment of the constriction within gastric band **102.** This will allow the clinician to observe the response of gastric band **102** to a patient's adjustment. This may permit a new modality for gastric band adjustment management to monitor pressure as well as volumes during adjustments. With these new pressure sensing capabilities, the clinician can make a determination of whether there is a leak within the system (e.g. zero pressure reading) or whether there is an obstruction in the system (e.g. prolonged pressure rise).

In an example embodiment, high precision pump unit **106** includes a first fluid line including a first pump for passing fluid in a first direction and a second fluid line in parallel with the first fluid line including a first valve and a second pump for passing fluid in an opposing direction. In another example embodiment, the second pump is not needed because pressure from the gastric band allows enough pressure the move the fluid to the reservoir. This use of parallel line configuration allows for filling and draining of gastric band with a minimal number of components and minimal complexity.

The systems and apparatus described herein can achieve at least one of the following features. The total time required to complete a fill or drain of gastric band **102** does not exceed about 10 minutes, more preferably about 5 minutes. The systems are able to adjust the volume in gastric band **102** accurately to within about 0.1 cm³ or about 10%, whichever is greater. Pressure/flow sensor 208 has a resolution between about 68.9 Pa (about 0.010 psi) to about 172 Pa (about 0.025 psi), preferably about 130 Pa (about0.019 psi).

In one example embodiment of the present description, components of the systems can be replaced without replacing the entire system and subjecting patients to overly invasive surgeries to replace entire systems when a single component is defective or damaged. For example, if high precision pump unit 106 becomes damaged, it can be replaced independently of other components. Alternatively, if gastric band 102 becomes damaged, it can be replaced independently of other components. The same is true of tubing 110 and reservoir 104. Although components can be disconnected for single part replacement, components shall not become dislodged from the tubing for tubing pu 11-off forces less than about 44.5 (about 10 Ibf), more preferably, less than about 22.2 N 5 lbf (5 lbf).

The systems described herein meet at least one safety specifications. For example, in the event of any failure of the systems, either no change in gastric band 102 tightness or a loosening of gastric band 102 results. Further, high precision pump unit 106 is biocompatible for long term implantation and remote controller unit 108 is biocompatible for transient use both per ISO 10993. The systems are designed to have no significant interaction or interference with other electronics in any of the following modalities: implantable energy sources such as defibrillators and pacemakers; internal energy sources such as electrosurgical instruments; external energy sources such as ultrasound, x-rays and defibrillators; and radiofrequency signals such as pacemaker programmers and neurostimulators.

### Example 1 Implantation of a Gastric Band System

A 40 year old female is diagnosed by her clinician as obese, weighing 231 kg (510 Ibs). The clinician suggests to the patient that she consider a gastric banding system according to the present description. She agrees and undergoes the implantation procedure. A gastric band is implanted around her cardia thereby creating a stoma. The high precision pump unit is sutured onto the rectus muscle sheath and the tubing and reservoir passes through the rectus muscle into the peritoneal cavity and connects to the gastric band. The system comes pre-filled, so there is no need for the clinician to fill the system during the surgical procedure. The patient is sutured and sent to recovery.

### Example 2 Adjustment of a Gastric Band System

The female patient of Example 1, after the completion of the surgical implantation, has her gastric band system properly adjusted by her clinician. The clinician holds a remote controller unit to the skin adjacent to the rectus muscle where the high precision pump unit is located and initiates communication between the devices. An initial pressure of zero is displayed for the gastric band as no fluid has been added to the gastric band. The clinician begins to fill the gastric band using saline housed within the reservoir at a rate of about 1 cm³/min and the entire filling takes less than about 5 minutes.

After filling, to about 137.9 Pa (about 20 psi), the patient is instructed to drink a glass of water in order to properly assess the proper inflation pressure of the gastric band to ensure it has not been over inflated. Upon confirmation that the gastric band is properly inflated, the procedure is completed and the patient returns to her normal life.

The patient instantly notices that she is much less hungry than she previously had been and is consistently consuming less food as her appetite has been decreased. She returns to her clinician's office for a follow-up visit three months after her implantation and initial gastric band filling and she has lost 9 kg (20 pounds). A year later, she has lost nearly 27 kg (60 Ibs).

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical value set forth in the specific examples are reported as precisely as possible. Any numerical value, however, inherently contains certain errors necessarily resulting from the standard deviation found in their respective testing measurements.

## Claims

1. A system (100) for treating obesity, the system comprising:
a gastric banding device (102) including an inflatable member (114) for containing fluid;
a fluid reservoir (104) couplable to the inflatable member(114);
an implantable pump (106) unit in communication with said fluid reservoir (104) and effective to control pressure within said inflatable member (114), said implantable pump unit including a inductively powered pump and a receiver assembly in communication with the pump; and
a remote control device (108) capable of communicating with the receiver assembly,
**characterized in that**
said pump unit (106) comprises a first fluid line including a unidirectional pump (206) for passing fluid in a first direction and a second fluid line in parallel with said first line including a valve (202) for passing fluid in an opposing direction.

2. The system of claim 1 wherein the pump (206) comprises a piezoelectric pump.

3. The system according to claim 1 or 2, wherein the pump (206) comprises a piezoelectric diaphragm pump.

4. The system according to any one of the preceding claims, wherein the pump (206) comprises a piezoelectric membrane.

5. The system according to any one of the preceding claims, wherein the pump (206) is a unidirectional pump.

6. The system according to any one of the preceding claims, wherein the implantable pump (206) unit is passive and includes no energy storage device.

7. The system according to any one of the preceding claims, wherein the implantable pump (206) unit is entirely inductively powered.

8. The system according to any one of the preceding claims, wherein the implantable pump unit (106) further comprises a pressure sensor (208) communicable with the remote control device (108).

9. The system according to any one of the preceding claims, wherein the implantable pump unit further comprises an access port (212) for enabling manual adjustment of a volume or a pressure of fluid in said inflatable member (114).

10. The system according to claim 9, wherein said access port (212) is an integral part of said pump unit (106).

11. The system according to any one of the preceding claims, wherein the implantable pump unit (106) further comprises at least one valve (204) in line with the pump (206).

12. The system according to claim 11, wherein the implantable pump unit further comprises another valve (202) in parallel with the pump.

13. The system according to any one of the preceding claims, further comprising:
a inductively powered, unidirectional piezoelectric diaphragm pump (206); and
an override port (212);
wherein the pump unit (206) being operable without the use of an implanted energy storage device.

14. The system according to any one of the preceding claims, comprising:
an external remote control device (108) capable of powering said implantable pump unit.

## Patentansprüche

1. System (100) zur Behandlung von Fettleibigkeit, wobei das System aufweist:
ein Magenband (102) aufweisend ein aufblasbares Element (114) zum Aufnehmen eines Fluides;
ein Fluidreservoir (104), das mit dem aufblasbaren Element (114) koppelbar ist;
eine implantierbare Pumpeneinheit (106) in Verbindung mit dem Fluidreservoir (104) und die in der Lage ist, einen Druck innerhalb des aufblasbaren Elementes (114) zu steuern, wobei die implantierbare Pumpeneinheit eine induktiv angetriebene Pumpe und eine Empfängeranordnung in Verbindung mit der Pumpe aufweist; und
eine Fernsteuereinrichtung (108), die in der Lage ist, mit der Empfängeranordnung in Verbindung zu stehen,
**dadurch gekennzeichnet, dass**
die Pumpeneinheit (106) eine erste Fluidleitung aufweisend eine unidirektionale Pumpe (206) zum hindurchlassen von Fluid in einer ersten Richtung und eine zweite Fluidleitung parallel zu der ersten Leitung aufweisend ein Ventil (202) zum Hindurchlassen von Fluid in einer entgegengesetzten Richtung umfasst.

2. System nach Anspruch 1, bei dem die Pumpe (206) eine piezoelektrische Pumpe aufweist.

3. System nach Anspruch 1 oder 2, bei dem die Pumpe (206) eine piezoelektrische Membranpumpe aufweist.

4. System nach einem der vorhergehenden Ansprüche, bei dem die Pumpe (206) eine piezoelektrische Membran aufweist.

5. System nach einem der vorhergehenden Ansprüche, bei dem die Pumpe (206) eine unidirektionale Pumpe ist.

6. System nach einem der vorhergehenden Ansprüche, bei dem die implantierbare Pumpeneinheit (206) passiv ist und keine Energiespeichereinrichtung aufweist.

7. System nach einem der vorhergehenden Ansprüche, bei dem die implantierbare Pumpeneinheit (206) vollständig induktiv angetrieben wird.

8. System nach einem der vorhergehenden Ansprüche, bei dem die implantierbare Pumpeneinheit (106) ferner einen Drucksensor (208), der mit der Fernsteuereinrichtung (108) in Verbindung bringbar ist, aufweist.

9. System nach einem der vorhergehenden Ansprüche, bei dem die implantierbare Pumpeneinheit ferner einen Zugriffsport (212) zum Ermöglichen einer manuellen Einstellung eines Volumens oder eines Drucks des Fluides in dem aufblasbaren Element (114) aufweist.

10. System nach Anspruch 9, bei dem der Zugriffsport (212) ein integrales Teil der Pumpeneinheit (106) ist.

11. System nach einem der vorhergehenden Ansprüche, bei dem die implantierbare Pumpeneinheit (106) ferner zumindest ein Ventil (204) in Reihe mit der Pumpe (206) aufweist.

12. System nach Anspruch 11, bei dem die implantierbare Pumpeneinheit ferner ein weiteres Ventil (202) parallel zu der Pumpe aufweist.

13. System nach einem der vorhergehenden Ansprüche, ferner aufweisend:
eine induktiv angetriebene, unidirektionale, piezoelektrische Membranenpumpe (206); und
einen Override-Anschluss (212);
wobei die Pumpeneinheit (206) ohne die Verwendung einer implantierten Energiespeichereinrichtung bedienbar ist.

14. System nach einem der vorhergehenden Ansprüche, aufweisend:
eine externe Fernsteuereinrichtung (108), die in der Lage ist, die implantierbare Pumpeneinheit mit Leistung zu beaufschlagen.

## Revendications

1. Système (100) pour traiter l'obésité, le système comprenant :
un dispositif de cerclage gastrique (102) comprenant un élément gonflable (114) pour contenir un fluide ;
un réservoir de fluide (104) pouvant être couplé à l'élément gonflable (114) ;
une unité de pompe implantable (106) en communication avec ledit réservoir de fluide (104) et efficace pour contrôler la pression dans ledit élément gonflable (114),
ladite unité de pompe implantable comprenant une pompe à alimentation inductive et un ensemble récepteur en communication avec la pompe ; et
un dispositif de commande à distance (108) capable de communiquer avec l'ensemble récepteur,
**caractérisé en ce que**
ladite unité de pompe (106) comprend une première conduite de fluide comprenant une pompe unidirectionnelle (206) pour faire passer un fluide dans une première direction et une deuxième conduite de fluide en parallèle avec ladite première conduite comprenant une vanne (202) pour faire passer le fluide dans une direction opposée.

2. Système selon la revendication 1, dans lequel la pompe (206) comprend une pompe piézoélectrique.

3. Système selon la revendication 1 ou 2, dans lequel la pompe (206) comprend une pompe à diaphragme piézoélectrique.

4. Système selon l'une quelconque des revendications précédentes, dans lequel la pompe (206) comprend une membrane piézoélectrique.

5. Système selon l'une quelconque des revendications précédentes, dans lequel la pompe (206) est une pompe unidirectionnelle.

6. Système selon l'une quelconque des revendications précédentes, dans lequel l'unité de pompe implantable (206) est passive et ne comprend pas de dispositif de stockage d'énergie.

7. Système selon l'une quelconque des revendications précédentes, dans lequel l'unité de pompe implantable (206) est entièrement à alimentation inductive.

8. Système selon l'une quelconque des revendications précédentes, dans lequel l'unité de pompe implantable (106) comprend en outre un capteur de pression (208) pouvant communiquer avec le dispositif de commande à distance (108).

9. Système selon l'une quelconque des revendications précédentes, dans lequel l'unité de pompe implantable comprend en outre un orifice d'accès (212) pour permettre un ajustement manuel d'un volume ou d'une pression de fluide dans ledit élément gonflable (114).

10. Système selon la revendication 9, dans lequel ledit orifice d'accès (212) fait partie intégrante de ladite unité de pompe (106).

11. Système selon l'une quelconque des revendications précédentes, dans lequel l'unité de pompe implantable (106) comprend en outre au moins une vanne (204) en ligne avec la pompe (206).

12. Système selon la revendication 11, dans lequel l'unité de pompe implantable comprend en outre une autre vanne (202) en parallèle avec la pompe.

13. Système selon l'une quelconque des revendications précédentes, comprenant en outre : une pompe à diaphragme piézoélectrique unidirectionnelle à alimentation inductive (206) ; et un orifice de contournement (212) ;
l'unité de pompe (206) pouvant fonctionner sans l'utilisation d'un dispositif de stockage d'énergie implanté.

14. Système selon l'une quelconque des revendications précédentes, comprenant :
un dispositif de commande à distance externe (108) pouvant alimenter ladite unité de pompe implantable.
